# EUROPEAN PATENT SPECIFICATION

(11) **EP 4 346 574 B1**
(45) Date of publication and mention of the grant of the patent: **25.03.2026**
(21) Application number: 22731516.5
(22) Date of filing: 25.05.2022
(51) Int. Cl.: A61B 5/0295, A61B 5/00

(54) **PERFUSION SHIFT MEASURING**
PERFUSIONSVERSCHIEBUNGSMESSUNG
MESURE DE DÉCALAGE DE PERFUSION

(30) Priority: 26.05.2021 US 202163193191 P
(43) Date of publication of application: 10.04.2024
(73) Proprietor: Koninklijke Philips N.V., 5656 AG Eindhoven (NL)
(72) Inventor: GROEN, Steven Peter, 5656 AG Eindhoven (NL); CANNON, Ryan, 5656 AG Eindhoven (NL)
(74) Representative: Philips Intellectual Property & Standards
(86) International application number: PCT/EP2022/064148
(87) International publication number: WO 2022/248528

(56) References cited:
- US-A1- 2010 331 708
- US-A1- 2015 282 748
- US-A1- 2018 042 494
- US-A1- 2018 132 736
- SEDLAK ET AL: "Hypothermia in trauma: The nurse's role in recognition, prevention, and management", ORAL SURGERY, ORAL MEDICINE, ORAL PATHOLOGY, ORAL RADIOLOGY AND ENDODONTICS, MOSBY-YEAR BOOK, ST. LOUIS, MO, US, vol. 1, no. 1, 1 January 1995 (1995-01-01), pages 19 - 26, XP005361423, ISSN: 1079-2104, DOI: 10.1016/S1079-2104(05)80406-8

## Description

### BACKGROUND

Human bodies require and regulate a precise and specific balance of oxygen in the blood. Oxygen saturation is the fraction of oxygen-saturated hemoglobin relative to total hemoglobin in the blood. Normal arterial blood oxygen saturation levels in humans are 95-100 percent, and can be measured by a SPO2 sensor which measures peripheral oxygen saturation (SPO2). The SPO2 sensor is a pulse oximeter sensor that passes small beams of light through blood at a location on the body to measure light absorption in the blood and to thereby measure the SPO2. A pulse oximeter is a monitor that analyzes measurements from the SPO2 sensor and provides a SPO2 level and may also provide a heart rate. Pulse oximeters may take readings from SPO2 sensors on or at a finger, a nasal ala, a forehead or an ear.

A nasal alar SPO2 sensor is a pulse oximeter sensor configured for use at the nasal ala and is used to monitor SPO2 in conjunction with a pulse oximeter. The nasal ala is the outer side of each nostril from above the nostril opening. The nasal ala is a location where an arterial plexus is present such that blood flow at the nasal ala is profound. The nasal alar SPO2 sensor includes a light receiver and two light emitting diodes, and may be provided with the pulse oximeter.

Another useful measurement in clinical settings is perfusion of blood. Perfusion of blood refers to the passage of blood through the circulatory system or lymphatic system, and usually refers to the delivery of blood to a capillary bed in tissue. Perfusion is measured as the rate at which blood is delivered to tissue, or volume of blood per unit time per unit tissue mass. As a person gets sick, the body of the person may shift perfusion by shifting blood away from the periphery and to the core organs such as the brain, heart and lungs to protect the core organs.

Hemodynamics are the dynamics of blood flow. Currently, demand for hemodynamic monitoring access is increasing so as to make the hemodynamic monitoring accessible to all clinicians. For example, non-invasive continuous blood pressure monitoring is a current research target. Continuous hemodynamic monitoring will allow for more strategic and timely interventions than does spot check monitoring, but will be expensive and require additional training and equipment.

Perfusion can be measured by cardiac output. An echocardiogram can measure cardiac output but is expensive, requires training in ultrasound, is typically only available in hospitals, takes an hour or more depending on clinician and equipment availability, is not continuous, and does not provide SPO2 measurements. While certain known devices are available to measure cardiac output, there are clear drawbacks such as performance, cost, safety and complexity

What is needed, therefore, is a perfusion shift measuring device that overcomes at least the drawbacks of known devices described above

Reference is made here to US 2018/132736 A1, which discloses a method that enables photoplethysmograph measurement of volume status. The disclosed method includes the steps of converting photoplethysmograph voltages to volume measurements and characterizing a local microcirculation as a microcosm in a manner allowing a photoplethysmograph to facilitate noninvasive monitoring of systemic status.

### SUMMARY

According to an aspect of the present disclosure, a system for monitoring a patient comprises: a first sensor adapted to be disposed at a central site, and adapted to provide a first signal for deriving perfusion index; a second sensor adapted to be disposed at a distal site, and adapted to provide a second signal for deriving perfusion index; a processor; a memory that stores instructions, which when executed by the processor, cause the processor to: correlate a first perfusion index based on the first signal from the first sensor and a second perfusion index based on the second signal from the second sensor, determine a trend based analysis of perfusion comprising a perfusion shift based on the correlated first perfusion index and second perfusion index, and determine the perfusion shift from a difference between a ratio of a current value of the first perfusion index to a current value of the second perfusion index, and a ratio of a past value of the first perfusion index to a past value of the second perfusion index.

According to another aspect of the present disclosure, a patient monitor comprises: a display and the system for monitoring a patient.

According to another aspect of the present disclosure, a tangible non-transitory computer readable storage medium that stores instructions, which, when executed by a processor, cause the processor to: correlate a first perfusion index based on the first signal from the first sensor and a second perfusion index based on the second signal from the second sensor, determine a trend based analysis of perfusion comprising a perfusion shift based on the correlated first perfusion index and second perfusion index, and determine the perfusion shift from a difference between a ratio of a current value of the first perfusion index to a current value of the second perfusion index, and a ratio of a past value of the first perfusion index to a past value of the second perfusion index.

According to another aspect of the present disclosure, a method for monitoring a patient is described. The method comprises: providing a first signal for deriving perfusion index from a first sensor adapted to be disposed at a central site; providing a second signal for deriving perfusion index from a second sensor adapted to be disposed at a distal site; correlating a first perfusion index based on the first signal from the first sensor and a second perfusion index based on the second signal from the second sensor, determining a trend based analysis of perfusion comprising a perfusion shift based on the correlated first perfusion index and second perfusion index, and determining the perfusion shift from a difference between a ratio of a current value of the first perfusion index to a current value of the second perfusion index, and a ratio of a past value of the first perfusion index to a past value of the second perfusion index. The present invention is directed to a system, storage medium and method as defined in the appended independent claims. Further details of the invention are defined in dependent claims.

### BRIEF DESCRIPTION OF THE DRAWINGS

The example embodiments are best understood from the following detailed description when read with the accompanying drawing figures. It is emphasized that the various features are not necessarily drawn to scale. In fact, the dimensions may be arbitrarily increased or decreased for clarity of discussion. Wherever applicable and practical, like reference numerals refer to like elements.
Fig.1 illustrates a system for determining a trend based analysis, in accordance with a representative embodiment.
Fig.2 is a flow-chart of a method for determining a trend based analysis, in accordance with a representative embodiment.
Fig. 3 illustrates a trend based analysis of a perfusion index and a cardiac output at selected location relative to a baseline perfusion index and cardiac output in accordance with a representative embodiment.
Fig. 4A illustrates a trend based analysis of perfusion indices versus time at selected location in accordance with a representative embodiment.
Fig. 4B illustrates a trend based analysis of cardiac output versus time at selected location in accordance with a representative embodiment.
Fig. 5A illustrates a display on a patient monitor showing a relative perfusion shift and a relative cardiac output in accordance with a representative embodiment.
Fig. 5B illustrates a display on a patient monitor showing a relative perfusion shift and a relative change in cardiac output in accordance with a representative embodiment.
Fig. 5C illustrates a display on a patient monitor showing a relative perfusion shift and a relative change in cardiac output in accordance with a representative embodiment.
Fig. 6 illustrates a display on a patient monitor showing a perfusion shift and a trending of change in cardiac output in accordance with a representative embodiment.

### DETAILED DESCRIPTION

In the following detailed description, for the purposes of explanation and not limitation, representative embodiments disclosing specific details are set forth in order to provide a thorough understanding of an embodiment according to the present teachings. Descriptions of known systems, devices, materials, methods of operation and methods of manufacture may be omitted so as to avoid obscuring the description of the representative embodiments. Nonetheless, systems, devices, materials and methods that are within the purview of one of ordinary skill in the art are within the scope of the present teachings and may be used in accordance with the representative embodiments. It is to be understood that the terminology used herein is for purposes of describing particular embodiments only and is not intended to be limiting. The defined terms are in addition to the technical and scientific meanings of the defined terms as commonly understood and accepted in the technical field of the present teachings.

It will be understood that, although the terms first, second, third etc. may be used herein to describe various elements or components, these elements or components should not be limited by these terms. These terms are only used to distinguish one element or component from another element or component. Thus, a first element or component discussed below could be termed a second element or component without departing from the teachings of the inventive concept.

The terminology used herein is for purposes of describing particular embodiments only and is not intended to be limiting. As used in the specification and appended claims, the singular forms of terms 'a', 'an' and 'the' are intended to include both singular and plural forms, unless the context clearly dictates otherwise. Additionally, the terms "comprises", and/or "comprising," and/or similar terms when used in this specification, specify the presence of stated features, elements, and/or components, but do not preclude the presence or addition of one or more other features, elements, components, and/or groups thereof. As used herein, the term "and/or" includes any and all combinations of one or more of the associated listed items.

Unless otherwise noted, when an element or component is said to be "connected to", "coupled to" another element or component, it will be understood that the element or component can be directly connected or coupled to the other element or component, or intervening elements or components may be present. That is, these and similar terms encompass cases where one or more intermediate elements or components may be employed to connect two elements or components. However, when an element or component is said to be "directly connected" to another element or component, this encompasses only cases where the two elements or components are connected to each other without any intermediate or intervening elements or components.

The present disclosure, through one or more of its various aspects, embodiments and/or specific features or sub-components, is thus intended to bring out one or more of the advantages as specifically noted below. For purposes of explanation and not limitation, example embodiments disclosing specific details are set forth in order to provide a thorough understanding of an embodiment according to the present teachings. However, other embodiments consistent with the present disclosure that depart from specific details disclosed herein remain within the scope of the appended claims. Moreover, descriptions of well-known apparatuses and methods may be omitted so as to not obscure the description of the example embodiments. Such methods and apparatuses are within the scope of the present disclosure.

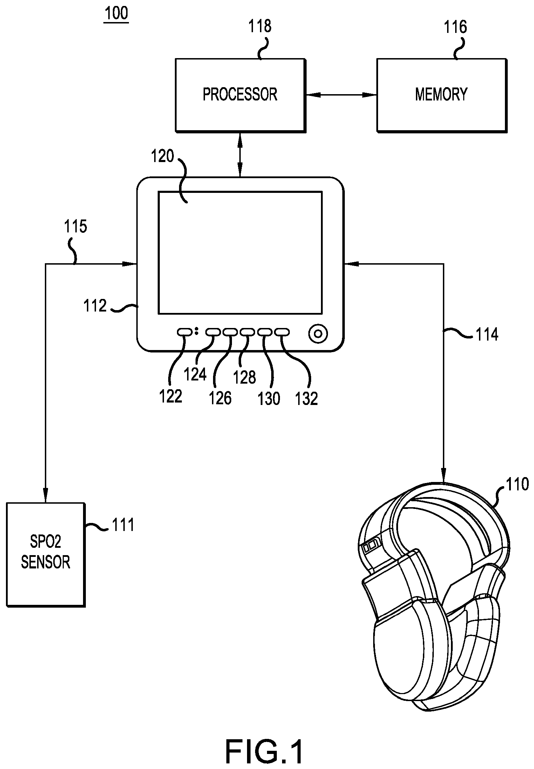

Fig.1 illustrates a system 100 for determining and displaying a trend based analysis, in accordance with a representative embodiment.

The system 100 in Fig.1 comprises a first sensor 110, a second sensor 111, a patient monitor 112, a first connection 114, a second connection 115, a processor 118 and a memory 116.

The first sensor 110 and the second sensor 111 are each adapted to measure oxygen saturation (SPO2) in a patient. Illustratively, the first sensor 110 is placed at a centrally perfused site ('central site') (e.g., nasal ala), and the second sensor 111 is placed at a distally perfused site ('distal site') (e.g., a finger). As is known, a pulse oximetry sensor is adapted to measure SPO2 by passing light through tissue at a location on the body such as a nasal ala to measure light absorption in the blood. As described more fully below, the memory 116 stores instructions, which when executed by the processor 118 are configured to determine a pulse rate, an SPO2 level, and a perfusion index at the sites where the first and second sensors 110, 111 are disposed on a patient.

During significant clinical events such as apneas and non-life-sustaining arrhythmias the body shifts blood away from the periphery and to the central organs such as the brain, heart and lungs. As described more fully below, comparisons of measurements from the first sensor 110 and the second sensor 111 will show a change in perfusion between central and peripheral sites, and at a specific time, or over a specified time period based on changes in pulsatility readings. The patient monitor 112 may be configured to continuously monitor hemodynamic characteristics by continuously correlating the first perfusion index and the second perfusion index based on the first signals from the first sensor 110 and the second signals from the second sensor 111.

Notably, as used herein, the perfusion index or perfusion numeric gives a value for the pulsatile portion of the measured signal (i.e., first and second signals from first and second sensors 110, 111) caused by the pulsating arterial blood flow in relation to the non-pulsatile portion of the measured signals. Stated somewhat differently, the perfusion index at particular sites on a patient (for example at a nasal ala and a finger) provide a measure of the pulsatility at the particular sites.

The first sensor 110 is illustratively an integrated sensor such as described in commonly owned Provisional Patent Application No. (Attorney Docket No. 2020P00800). The first sensor 110 is adapted to measure a variety of vital signs of a patient. For example, the first sensor 110 is adapted to measure oxygen saturation (pulse oximetry), the temperature of ambient air at the nasal ala, the temperature of exhaled air exiting a nasal passage at the application site, and the temperature of the skin (e.g., on the cheek), and pulsatility. As will become clearer as the present description continues, the first sensor 110 provides first signals

Various aspects of the pulse oximetry sensor of the first sensor 110 are described, for example, in U.S. Patent 10,390,715.

The second sensor 111 illustratively is an SPO2 sensor adapted to measure oxygen saturation at a distally perfused site.

As described more fully below, the first connection 114 and the second connection 115 each may be electrical cables that connect the first sensor 110 and the second sensor 111, respectively, to the patient monitor 112. Generally, the cable comprises a signal transmission line and a power line. Notably, the use of an electrical cable for first and second connections 114, 115 is merely illustrative, and other types of connections are contemplated to provide communication between the first and second sensors 110, 111 and the patient monitor 140. By way of illustration, instead of the electrical cable, the first and second connections 114, 115 may be via an optical fiber transmission line. Alternatively, the first and second connections 114, 115 between first and second sensors 110, 111, respectively, and the patient monitor 140 may be wireless. Just by way of illustration, a wireless link compliant with IEEE 802.11(x) is contemplated to effect the connection between the first and second sensors 110, 111 and the patient monitor 140.

To measure temperature, when functioning as the integrated sensor described in the copending provisional application, the first sensor 110 must receive electrical power. As such, while optical fiber and wireless communication are contemplated for transmission of data between the patient monitor 140 and the first and second sensors 110, 111, first connection 114 must also provide power for operation of the first sensor 110. In certain embodiments, the transmission of power from the patient monitor 140 may be effected by a separate power line connected between the patient monitor 140 and the first sensor 110. Moreover, the present teachings contemplate a separate (portable) device comprising a battery to connect to the first sensor 110. In such a representative embodiment, the separate device may be held or attached to a patient providing power to the first sensor 110. Data from the first sensor 110 could then be transmitted wirelessly from a transmitter disposed in the first sensor 110 or from the separate device. Alternatively, for example when the first connection 114 is wireless, the present teachings contemplate one of a number of wireless power techniques (WPTs) that are near field (within approximately one wavelength (*λ*) of the antenna of the transmission system), or far field at a distance greater than approximately one wavelength (*λ*) of the antenna of the transmission system.

In combination, the memory 116 and processor 118 may be referred to as a controller. In certain representative embodiments, the memory 116 and the processor 118 may be components disposed in the patient monitor 112. Alternatively, the memory 116 and the processor 118 may be separate elements from the patient monitor 112. Just by way of illustration, the first and second connections 114 may comprise a Smart Cable (e.g., serial RS232 cable), which in-turn comprises the memory 116 and the processor 118.

The system 100 can be deployed as, or in, a device that in turn is in an integrated system that includes additional devices. In an embodiment, the system 100 can be implemented using electronic devices that provide voice, video or data communication. The system 100 may be a self-contained system, or more generally, may be deployed as a part of a computer network. In this case, the processor 118 and memory 116 may be components of a server or as a client user computer in a server-client user network environment, or as a peer computer system in a peer-to-peer (or distributed) network environment. Further, while the system 100 is illustrated in the singular, the term "system" shall also be taken to include any collection of systems or subsystems that individually or jointly execute a set, or multiple sets, of software instructions to perform one or more computer functions.

The patient monitor 112 comprises a display 120. The display 120 may be one of a number of known types of displays, including but not limited to a light emitting diode (LED), a liquid crystal display (LCD), an organic light emitting diode (OLED), a flat panel display, a solid-state display, or a cathode ray tube (CRT), for example. Notably, and as will be described more fully below, in accordance with various representative embodiments, the display 120 is adapted to show a trend based analysis, including certain medical information determined by execution of instructions stored in the memory 116, and by the processor 118, to include a perfusion index at a central (or core) site (PerfC), a perfusion index at a distal site (PerfD), a Cardiac Output (CO), and beneficially a trend based analysis either relative to immediately preceding measurements (e.g., 30 minutes prior) and current measurements, or a trend based analysis of the PerfC, PerfD, Cardiac Output, and beneficially a trend based analysis over a specified time duration (e.g., twelve hours).

The patient monitor 112 also comprises at least a first interface 122, a second interface 124, a third interface 126, a fourth interface 128, a fifth interface 130 and sixth interface 132. The various interfaces are selected for specific functions useful in the gathering of data from the first sensor 110. For example, one of the interfaces may be a Fourier artifact suppression technology (FAST) socket used to connect cables (in first connection 114 and second connection 115) to a circuit board of the patient monitor 112 such as an SPO2 circuit board. More generally, the first~sixth interfaces 122-132 may be configured to connect a variety of other electronic devices to the patient monitor 140.

The patient monitor 112 may comprise a stationary computer, a mobile computer, a personal computer (PC), a laptop computer, a tablet computer, a mobile/cellular telephone, or any other machine capable of executing a set of software instructions (sequential or otherwise) that specify actions to be taken by that machine.

The processor 118, which is tangible and non-transitory, is representative of one or more processors. As used herein, the term "non-transitory" is to be interpreted not as an eternal characteristic of a state, but as a characteristic of a state that will last for a period. The term "non-transitory" specifically disavows fleeting characteristics such as characteristics of a carrier wave or signal or other forms that exist only transitorily in any place at any time. The processor 118 (and other processors) of the present teachings is an article of manufacture and/or a machine component.

The processor 118 is configured to execute software instructions stored in the memory 116 to perform functions as described in the various embodiments herein. The processor 118 may be a general-purpose processor or may be part of an application specific integrated circuit (ASIC). The processor 118 may also be (or include) a microprocessor, a microcomputer, a processor chip, a controller, a microcontroller, a digital signal processor (DSP), a state machine, or a programmable logic device. The processor 118 may also be (or include) a logical circuit, including a programmable gate array (PGA) such as a FPGA, or another type of circuit that includes discrete gate and/or transistor logic. The processor 118 may be (or include) a central processing unit (CPU), a graphics processing unit (GPU), or both. Additionally, the processor 118 may comprise multiple processors, parallel processors, or both. Multiple processors may be included in, or coupled to, a single device or multiple devices.

The memory 116 may comprise a main memory, a static memory, or both, where the memories may communicate with each other via a bus (not shown). The memory 116 described herein are tangible storage mediums that can store data and executable instructions, and are non-transitory during the time instructions are stored therein. As used herein, the term "non-transitory" is to be interpreted not as an eternal characteristic of a state, but as a characteristic of a state that will last for a period. The term "non-transitory" specifically disavows fleeting characteristics such as characteristics of a carrier wave or signal or other forms that exist only transitorily in any place at any time.

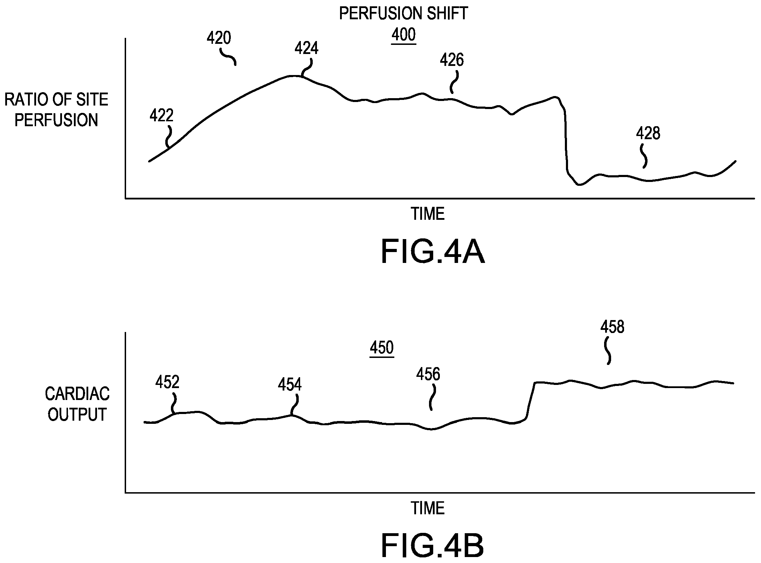

The memory 116 of the present teachings is an article of manufacture and/or machine component. The memory 116 includes one or more computer-readable mediums from which data and executable instructions (e.g., to carry out the processes described in connections with Fig. 4) can be read by a computer. Memories as described herein may be random access memory (RAM), read only memory (ROM), flash memory, electrically programmable read only memory (EPROM), electrically erasable programmable read-only memory (EEPROM), registers, a hard disk, a removable disk, tape, compact disk read only memory (CD-ROM), digital versatile disk (DVD), floppy disk, Blu-ray disk, or any other form of storage medium known to one of ordinary skill in the art. Memories of the present teachings may be volatile or non-volatile, secure and/or encrypted, unsecure and/or unencrypted. The patient monitor 112, the memory 116 and the processor 118 may be housed within or linked to a workstation (not shown) such as a computer or another assembly of one or more computing devices, a display/monitor, and one or more input devices (e.g., a keyboard, joysticks and mouse) in the form of a standalone computing system, a desktop or a tablet, for example.

The memory 116 stores instructions that are executed by the processor 118 to implement aspects of methods described herein. For example, the processor 118 may apply instructions to the measurements received from respective light receivers (not shown) of the first and second sensors 110, 111, and temperature sensor(s) of the first sensor 110. The processor 118 may create time series of measurements and generate displays of the time series for the display 120.

The instructions stored in the memory 116 and executed by the processor 118 may include one or more software programs used to display time series including time series of measurements captured by the first sensor 110. For example, the circuit board of the patient monitor 112 may be configured to convert analog signals of the oxygen saturation to digital signals, and the processor 118 may be configured to process the digital signals of the oxygen saturation by executing the instructions to generate the time series and other types of information. Additionally, the circuit board of the patient monitor 112 may be configured to convert analog signals of the temperatures to digital signals, and the processor 118 may be configured to process the digital signals of the temperatures by executing the instructions to generate the time series and other types of information.

Among other functions, and as described more fully below, the processor 118 may be configured to determine one of a variety of useful measurements of a patient based on measurements made by the first and second sensors 110, 111.

As described above, the first sensor 110 may include a nasal alar pulse oximetry sensor worn on a nasal ala by a patient. The second sensor 111 may be a finger pulse oximetry sensor worn on a finger by the patient. The first and second connections 114, 115 may each be compatible SpO2 adapter cables. The patient monitor 112 is adapted to measuring SPO2 signals from the first and second sensors 110, 111 at once. An example of such a patient monitor 112 is an IntelliVue^{®} series monitor from Koninklijke Philips N.V modified to carry out the various functions of the system 100 of the present teaching.

As described more fully below, the processor 118 is configured to execute instructions stored in the memory to determine, inter alia, perfusion indices and a trend based analysis for the locations of the first and second sensors 110, 111.

As described more fully below, a first perfusion index and a second perfusion index are correlated using a known method (e.g., a Fourier artifact suppression technology (FAST) algorithm, a Rainbow algorithm, or a Signal Extraction Technology (SET) algorithm) stored as executable instructions in memory 116. The FAST, Rainbow, or SET algorithms, which are executed by the processor 118, receive as an input on a first signal from the first sensor 110 and a second signal from a second sensor 111, and provide as an output, the perfusion index for the particular sites of the first and second sensors 110, 111.

Continuing the present illustrative application, the processor 118 correlates the first and second perfusion indices and determines a trend based analysis of perfusion between the two sites (e.g., central and peripheral) locations of the body where measurements are made. This trend based analysis beneficially provides an indication in any shift in the perfusion index (e.g., more central or core perfusion) compared to a recent measure of the perfusion indices (baseline indices), or compared to ratios of site perfusion over time. Just by way of illustration, a clinician may wish to determine the impact of an oral medication on a patient's perfusion at particular locations of the body. This impact may take approximately 30 min. As described more fully below, in accordance with a representative embodiment, the current perfusion indices and cardiac output may serve as baseline readings for a patient. The processor 118 may execute instructions stored in the memory 116 to determine the perfusion indices at the selected locations at a later time (e.g., 30 minutes later) and compare them to the baseline perfusion indices. From this comparison, a trend based analysis comprising a variable ratio of the perfusion indices can be determined. Specifically, the variable ratio provides a measure of the change in the balance between central and peripheral perfusion. Moreover, the processor may also determine trend changes in cardiac output based on the perfusion of two discreet tissues at central and peripheral sites, which may also be included in the trend based analysis.

Alternatively or additionally, and as described more fully below, the processor 118 may execute instructions stored in the memory 116 to determine ratio of perfusion at discreet central and peripheral sites (e.g., PerfC: PerfD), or the relative cardiac output change, or both, at the selected locations over a selected period of time. For example, it may be useful to study changes in the perfusion indices based on first and second signals from the first and second sensors 110, 111, respectively, or the relative cardiac output, or both, over a period of time where a number of changes in treatment may occur. In such an example setting, the impact of the various treatments on the perfusion indices at the selected locations, or the cardiac output trend, or both, over time can be studied to determine their impact on the patient. Just by way of illustration, it may be useful for a practitioner to have a histogram showing the ratio of perfusion indices at the selected locations of the first and second sensors 110, 111, or the cardiac output, or both, over a twelve (12) hour window of time to see what impact the intervening treatments had on the patient.

The memory 116 may also store and execute another algorithm to determine perfusion shifts and changes in cardiac output or perfusion. The detection algorithm used to detect perfusion shifts is included in as executable instructions (a software program) stored in the memory 116 and executed by the processor 118 to detect perfusion shift from the time series. The detection algorithm is implemented by a computer program of instructions executed by the processor 118. When executed by the processor 118, the instructions cause the processor 118 to correlate a first perfusion index based on first signals from the first sensor 110 to a second perfusion index based on second signals from the second sensor 111. The instructions cause the processor 118 to determine a trend in change in perfusion index, or cardiac output, or both, between central and peripheral locations based on the first signals and on the second signals, and to determine the relative cardiac output. These data may then be used to present a trend based analysis of perfusion index, or relative cardiac output, or both, at specific locations (central and peripheral) of the body a specific time, and display these data relative to an earlier (e.g., 30 min) perfusion index, or cardiac input, or both (sometimes referred to as baseline data) on the display 120 of the patient monitor. Notably, the cardiac output is inferred based on the change of the relationship between the central and peripheral sites relative to a previous reading. Alternatively, or additionally, these instructions cause the processor 118 to determine a perfusion indices at a specific location ("site index") of the body (e.g., PerfC, PerfD), ratios of site perfusion indices (e.g., PerfC: PerfD), or cardiac output, or both, over a specified period of time (e.g., 12 hours). These data may then be used to present a trend based analysis on the display 120 of the patient monitor.

When the processor 118 executes the instructions, the instructions may cause the processor 118 to determine a change in the perfusion index at specific sites (e.g., central and peripheral) and at a specific time in multiple different ways. For example, perfusion index may be determined from the first signals and the second signals based on comparing current values of the first signals to past values of the first signals and comparing current values of the second signals to past values of the second signals. Additionally or alternatively, the change in perfusion index at specific sites (e.g., central and peripheral) and at a specific time may be determined from the first signals and the second signals based on comparing differences between current values of the first signals and past values of the first signals to differences between current values of the second signals and past values of the second signals. The comparisons may be between individual current values and individual past values, or averages of multiple current and recent values and multiple past values. The comparisons may also be based on medians of multiple current and recent values and medians of multiple past values.

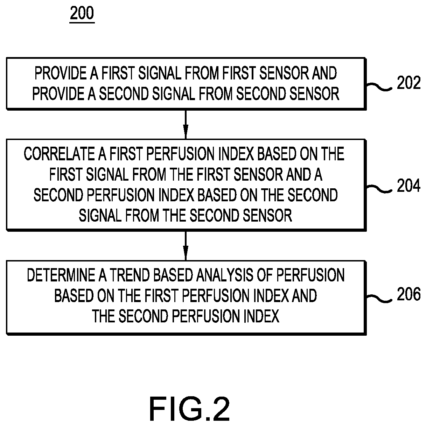

Fig. 2 is a flow-chart of a method 200 for determining a trend based analysis, in accordance with a representative embodiment. The method 200 is contemplated to be implemented in system 100, or in patient monitor 112, or stored as executable instructions on a tangible non-transitory computer readable storage medium (e.g., memory 116). When executed by a processor (e.g., 118), the instructions cause the processor to perform the method. Accordingly, various details and aspects of the system 100 described above are common to the representative embodiments described in connection with Fig. 2, and may not be repeated to avoid obscuring the description of the presently described representative embodiments.

At 201 a first signal from a first sensor and a second signal from a second sensor are provided. Illustratively, the first signal is from a central site, and is provided from first sensor 110 to the patient monitor 112 via the first connection 114; and the second signal is from a peripheral site, and is provided from second sensor 111 as described above.

At 202, a first perfusion index, based on the first signal from the first sensor is correlated. Similarly, a second perfusion index, based on the second signal from the second sensor is correlated. Illustratively, the correlation of the first and second perfusion indices is effected using a known method (e.g., a FAST algorithm or a SET algorithm) stored as executable instructions in memory 116. The FAST or SET algorithms, which are executed by the processor 118, receive as an input the first signal from the first sensor 110 and the second signal from the second sensor 111, and provide as an output, the perfusion index for the particular sites of the first and second sensors 110, 111. Continuing this illustrative example, with the first signal's being correlated to provide PerfC and the second signal's being correlated to provide PerfD.

At 206 a trend based analysis of perfusion is determined based on the first perfusion index and the second perfusion index. As alluded to above, and as described more fully below, in accordance with a representative embodiment, the trend based analysis may include certain medical information determined by execution of instructions stored in the memory 116, and by the processor 118. These medical information illustratively comprise include PerfC, PerfD, and CO. The trend based analysis of the PerfC, PerfD, and CO, which may be provided to the display 120 for example, is either relative to immediately preceding measurements (e.g., 30 minutes prior) and current measurements, or over a specified time duration (e.g., twelve hours).

Notably, in accordance with a representative embodiment, a change in the perfusion index between a central site and a peripheral site is determined by determining a difference between a ratio of the perfusion indices at the central site and the peripheral site at a current time (PerfC2:PerfD2), and a ratio of the perfusion indices at the central site and the peripheral site at an earlier time (PerfC1:PerfD1). As described more fully below, this change in the perfusion index may show a shift in perfusion from the central (core) portion of a patient to the peripheral (distal) site, or vice versa. As noted above, these changes/shifts in perfusion can be presented on display 120 relative to a recent baseline. Alternatively, or additionally, ratios of site perfusion (PerfC: PerfD), or CO, or both, at the selected locations over a selected period of can be displayed over time.

Moreover, in accordance with a representative embodiment, a change in the cardiac output is determined a difference of a sum of a current value of the first perfusion index and a current value of the second perfusion index (PerfC2+PerfD2), and a sum of a past value of the first perfusion index and a past value of the second perfusion index (PerfC1+PerfD1). As described more fully below, the change in the CO can be presented on display 120 relative to a recent baseline, or the relative CO over time can be displayed over time.

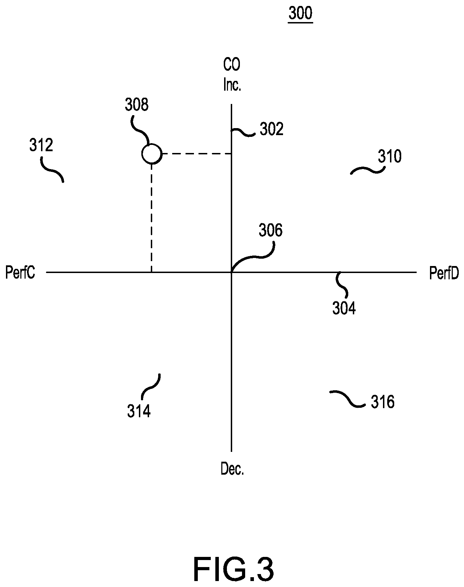

Fig. 3 illustrates a trend based analysis 300 of a perfusion index and a cardiac output at selected location relative to a baseline perfusion index and cardiac output in accordance with a representative embodiment. Illustratively, the trend based analysis 300 comprises data determined using the system 100 and method 200, and is contemplated for displaying on display 120. Various details and aspects of the trend based analysis of the representative embodiments described in connection with Fig. 3 are common to those of the representative embodiments described above in connection with Figs. 1 and 2, and may not be repeated to avoid obscuring the description of the presently described representative embodiments.

As described more fully below, the trend based analysis 300 provides a measure of changes in perfusion and cardiac output compared to most-recent ("baseline") measurements of perfusion and cardiac input.

With reference to Fig. 3, CO data are plotted along an ordinate 302 with an increase in CO being above an origin 306, and a decrease in CO below the origin 306. Similarly, perfusion indices at central and distal sites (PerfC, PerfD) are plotted along abscissa 304, with PerfD to the right of the origin 306 and PerfC to the left of the origin 306.

In accordance with a representative embodiment, the origin 306 represents a baseline value of correlated perfusion indices and relative CO. Continuing the illustrative example, clinician may wish to determine the impact of an oral medication on a patient's perfusion at particular locations of the body. This impact may take approximately 30 min. As described more fully below, in accordance with a representative embodiment, the previous (e.g., 30 minutes before) perfusion indices and cardiac output serve as baseline readings for a patient, and are plotted at the origin 306. The processor 118 may execute instructions stored in the memory 116 to determine the perfusion indices at the selected locations at a current time (e.g., 30 minutes after administering the oral medication) and compare them to the baseline perfusion indices. From this comparison, a trend based analysis comprising a shift in the perfusion indices can be determined.

In the illustrative example depicted in Fig. 3, a current reading is plotted at 308. As such, relative to the origin, the perfusion has shifted toward the core, and the CO has increased compared to the measurements at the time of administering the oral medication.

More generally, measurements plotted in a first quadrant 310 show in increase in perfusion at the distal site (and away from the central site) and an increase in CO relative to the baseline readings of perfusion and CO at a selected time duration (e.g., 30 minutes) earlier.

Measurements plotted in a second quadrant 312 show in increase in perfusion at the central site (and away from the distal site) and an increase in CO relative to the baseline readings of perfusion and CO at a selected time duration (e.g., 30 minutes) earlier.

Measurements plotted in a third quadrant 314 show in a decrease in perfusion at the central site (and toward the distal site) and a decrease in CO relative to the baseline readings of perfusion and CO at a selected time duration (e.g., 30 minutes) earlier.

Measurements plotted in a fourth quadrant 316 show in a decrease in perfusion at the distal site (and toward the central site) and a decrease in CO relative to the baseline readings of perfusion and CO at a selected time duration (e.g., 30 minutes) earlier.

Fig. 4A illustrates a trend based analysis 400 of perfusion indices versus time at selected locations in accordance with a representative embodiment. Illustratively, the trend based analysis 400 comprises data determined using the system 100 and method 200, and is contemplated for displaying on display 120. Various details and aspects of the trend based analysis of the representative embodiments described in connection with Fig. 4A are common to those of the representative embodiments described above in connection with Figs. 1-3, and may not be repeated to avoid obscuring the description of the presently described representative embodiments.

Referring to Fig. 4A, the trend based analysis 400 shows a ratio of site perfusion (e.g. PerfC: PerfD) of a patient over time. Notably, and as described above, in accordance with a representative embodiment, the executable instructions stored in memory 116 cause the processor 118 to determine perfusion indices at a specific location ("site index") of the body (e.g., PerfC, PerfD), and ratios of site perfusion indices (e.g., PerfC: PerfD) over a specified period of time (e.g., 12 hours). These data may then be used to present a trend based analysis on the display 120 of the patient monitor 112.

As noted above, perfusion shift 420 beneficially is provided as a histogram showing the ratio of perfusion indices at the selected locations of the first and second sensors 110, 111 over a twelve (12) hour window of time to see what impact the intervening treatments had on the patient. For example, suppose a review of the trend based analysis 400 provides a ratio of the central site perfusion index to the distal site perfusion index (PerfC: PerfD) over time. At a particular time, for example at 424, the trend based analysis 400 shows the perfusion at the central site is increased relative to an earlier time 422. In region 426, the perfusion at central site has decreased compared to its value at 424, but remains increased relative to the earlier time 422. Finally, in region 428, the perfusion at the central site is decreased significantly, and is now decreased relative to earlier times 422, 424.

As such, the trend based analysis 400 shows the clinician that initially the shift in perfusion was from the peripheral site to the central site indicating that blood has flowed to the core in the time between 422 and 424. This shift could be caused by an acute event that caused the body of the person to shift perfusion by shifting blood away from the periphery and to the core organs such as the brain, heart and lungs to protect the core organs.

Similarly, the trend based analysis 400 shows there is comparatively little shift in the blood flow in region 426, where the ratio of the site indices are comparatively unchanged.

Finally, the trend based analysis 400 shows the clinician in region 428 that the perfusion has shifted away from central perfusion to more peripheral perfusion. As such, in region 428, an acute event caused the body of the person to shift perfusion by shifting blood away from the core organs and to the periphery (e.g., the legs and arms).

Beneficially, the trend based analysis 400 along with a record of various medical treatments and/or external factors can show how these treatments and/or factors have impacted blood perfusion in the patient.

Fig. 4B illustrates a trend based analysis 450 of cardiac output versus time at selected location in accordance with a representative embodiment. Illustratively, the trend based analysis 400 comprises data determined using the system 100 and method 200, and is contemplated for displaying on display 120. Various details and aspects of the trend based analysis of the representative embodiments described in connection with Fig. 4B are common to those of the representative embodiments described above in connection with Figs. 1 and 2, and may not be repeated to avoid obscuring the description of the presently described representative embodiments.

The trend based analysis 450 beneficially provides a histogram showing the CO at the selected locations of the first and second sensors 110, 111 over a twelve (12) hour window of time to see what impact the intervening treatments had on the patient. At a time in the trend based analysis, for example at 454, the trend based analysis shows the CO is increased slightly relative to an earlier time 452. In region 456, the CO is relatively unchanged. Finally, in region 428, the CO has increased significantly, and is now increased relative to earlier times 452, 454.

Notably, in accordance with a representative embodiment, the trend based analysis 450 begins and ends at the same time as the trend based analysis 400. As such, for each point in time, the clinician can review (on display 120, for example) the CO and the ratio of the central site perfusion index to the distal site perfusion index (PerfC: PerfD).

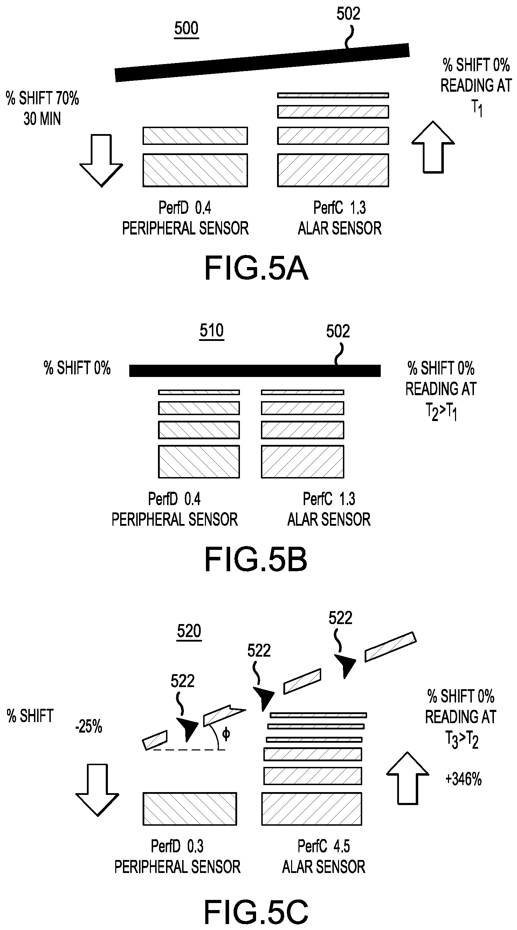

Fig. 5A illustrates a trend based analysis 500 including a perfusion shift at time T1 to be provided on a display (e.g., display 120) in accordance with a representative embodiment. Various details and aspects of the display of the perfusion shift of the representative embodiments described in connection with Fig. 5A are common to those of the representative embodiments described above in connection with Figs. 1, 2, 4A and 4B, and may not be repeated to avoid obscuring the description of the presently described representative embodiments.

The display depicted in Fig. 5A shows PerfC based on measurements from the first sensor 110, and PerfD based on measurements from the second sensor 111. The arrows indicate the relative historical shift at each sensor.

In Fig. 5A, the PerfD measured at T1 by the second sensor 111 has decreased compared to an earlier time (e.g., 30 minutes prior), and the PerfC measured by the first sensor 110 has increased compared to the earlier time. Specifically, based on measurements of the first sensor 110, the perfusion shift at the central site is + 30%, whereas the perfusion shift based on measurements from the second sensor 111 is -70%. Moreover, the PerfD is approximately 0.4 at T1, and the PerfC is approximately 1.3 at T1.

Finally, based on measurements taken by the first and second sensors 110, 111, the CO 502 has increased slightly at T1.

The display depicted in Fig. 5A shows PerfC based on measurements from the first sensor 110, and PerfD based on measurements from the second sensor 111. The arrows indicate the relative historical shift at each sensor.

Fig. 5B illustrates a trend based analysis 500 including a perfusion shift at time T2 (>T1) to be provided a display (e.g., display 120) accordance with a representative embodiment. Various details and aspects of the display of the perfusion shift of the representative embodiments described in connection with Fig. 5B are common to those of the representative embodiments described above in connection with Figs. 1, 2, and 4A-5A, and may not be repeated to avoid obscuring the description of the presently described representative embodiments.

The display depicted in Fig. 5B shows PerfC based on measurements from the first sensor 110, and PerfD based on measurements from the second sensor 111.

In Fig. 5B, the PerfD measured at T2 by the second sensor 111 has remained constant compared to its value at T1, and the PerfC measured by the first sensor 110 also remained constant compared to its value at T1. Specifically, based on measurements of the first sensor 110, the perfusion shift at the central site is 0%, whereas the perfusion shift based on measurements from the second sensor 111 is 0%. As such, the PerfD remains approximately 0.4 at T2, and the PerfC remains approximately 1.3 at T2.

Finally, based on measurements taken by the first and second sensors 110, 111, the CO 502 remains unchanged at T2 (compared to its value at T1).

Fig. 5C illustrates a trend based analysis 520 including a perfusion shift at time T3 (>T2) to be provided a display (e.g., display 120) accordance with a representative embodiment. Various details and aspects of the display of the perfusion shift of the representative embodiments described in connection with Fig. 5B are common to those of the representative embodiments described above in connection with Figs. 1, 2, and 4A-5B, and may not be repeated to avoid obscuring the description of the presently described representative embodiments.

The display depicted in Fig. 5C shows PerfC based on measurements from the first sensor 110, and PerfD based on measurements from the second sensor 111. The arrows indicate the relative historical shift at each sensor.

In Fig. 5C, the PerfD measured at T3 (>T2) by the second sensor 111 has decreased compared to an earlier time (e.g., 30 minutes prior), and the PerfC measured by the first sensor 110 has increased compared to the earlier time. Specifically, based on measurements of the first sensor 110, the perfusion shift at the central site is + 346%, whereas the perfusion shift based on measurements from the second sensor 111 is -25%. Moreover, the PerfD is approximately 0.4 at T3, and the PerfC is approximately 1.3 at T3.

Arrows 522 show the change in CO over time based on measurements taken by the first and second sensors 110, 111. The change in the angle φ indicates the degree of change of perfusion at T3 compared to its value at T2. So, φ= 0° would indicate perfusion (pulsatility) at the central and peripheral sites is unchanged from the baseline, whereas a comparatively steep angle indicating that the balance of the perfusion has shifted centrally or peripherally (in this case from peripheral to central). For purposes of illustration, this can be described numerically as a contributing percentage to the total sum of the perfusion indices (the cardiac output metric). In Fig. 5B, the peripheral sensor contributes about 25% to the sum of the perfusion indices. In 5C Fig. 5B, the peripheral sensor contributes about 6% of the 19% decrease in contribution constitutes a centralization. More generally, the shift percentages provide a comparison of the change in perfusion against its own baseline, whereas the angle φ of the top line indicates change in its proportion to the total sum of the perfusion indices.

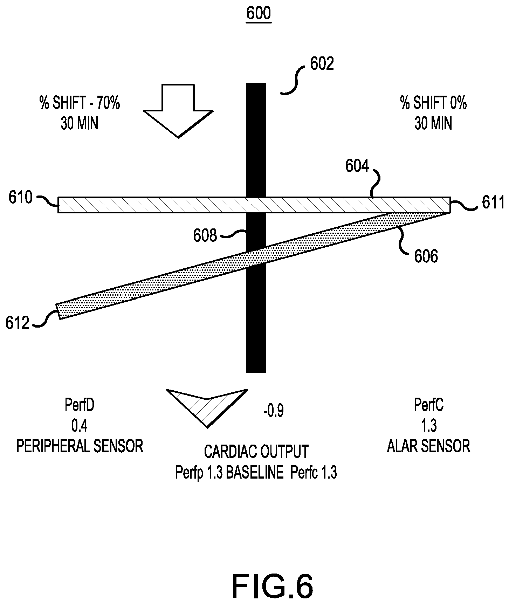

Fig. 6 illustrates a trend based analysis 600 including a perfusion shift over a time interval (e.g., 30 minutes) to be provided to a display (e.g., display 120) accordance with a representative embodiment. Various details and aspects of the display of the perfusion shift of the representative embodiments described in connection with Fig. 5B are common to those of the representative embodiments described above in connection with Figs. 1, 2, and 4A-5A, and may not be repeated to avoid obscuring the description of the presently described representative embodiments.

The vertical line 602 plots the CO at a particular time, and first and second lines 604, 606 depict the perfusion shift. Specifically, the intersection of the first line 604 with the vertical line 602 depicts the CO at a first time T1, and the intersection of the second line 606 with the vertical line depicts the CO at a second time T2, after the first time. The difference 608 between these points of intersection shows the magnitude of the change of the CO between T1 and T2. Notably, the CO at T1 and at T2 are determined based as described above. Illustratively, between T1 and T2, the CO of the patient has decreased by 0.9.

The trend based analysis 600 depicts PerfD to the left of the vertical line 602 and PerfC to the right of the vertical line 602. At T1, the PerfD is at 610 and the PerfC is at 611. At time T2, however, the PerfD has decreased to 612, whereas the PerfC remains at 611. Accordingly, the PerfD has decreased, and the PerfC has remained constant. Illustratively, the PerfD has decreased from 1.3 to 0.9, resulting in a shift of approximately 70% between T1 and T2 (illustratively 30 minutes). By contrast, the PerfC remains at 1.3 and there is no shift in perfusion measured at the central location (e.g., measured by the second sensor 111).

Beneficially, the trend based analysis 600 allows a clinician to review changes in PerfC, PerfD and CO over time. Notably, in the depicted embodiment, the readings at vertical line 602 can be set as the baseline, and the readings at 604 at a later time, much like the trend based analysis discussed above, for example in connection with the representative embodiments of Fig. 3.

In some embodiments, perfusion shifts from periods may be recorded and then made selectable for comparisons. For example, if a clinician wants to compare a period when a medication was administered before with a current period when the medication is again administered, the perfusion values from the prior period may be compared and made visually displayable with the current period. In some embodiments, perfusion values from more than three periods may be compared and made visually displayable, such as to show how perfusion shifts are changing over multiple time periods.

In the interface in Fig.6, the perfusion measured by the second sensor 111 is decreasing compared to 30 minutes prior, and the perforation measured by the nasal alar (core) sensor is increasing compared to 30 minutes prior. The shift measured by the second sensor 111 is shown as a 70% decrease. The absolute current value measured by the second sensor 111 is shown as .4, and the absolute current value measured by the nasal alar (core) sensor is shown as 1.3. Additionally, a trend in cardiac output for the current period is shown to be increasing from lower left to upper right, but the comparison with the previous period is shown to be a decrease of 0.9.

The inventive concepts also encompass a computer readable medium that stores instructions that cause a data processing system (such as the processor 118) to execute the methods described herein. A computer readable medium is defined to be any medium that constitutes patentable subject matter under 35 U.S.C. §101 and excludes any medium that does not constitute patentable subject matter under 35 U.S.C. §101. Examples of such media include non-transitory media such as computer memory devices that store information in a format that is readable by a computer or data processing system. More specific examples of non-transitory media are noted above.

Although trend based analyses have been described with reference to several representative embodiments, it is understood that the words that have been used are words of description and illustration, rather than words of limitation. Changes may be made within the purview of the appended claims, as presently stated and as amended, without departing from the scope and spirit of perfusion shift measuring in its aspects. Although perfusion shift measuring has been described with reference to particular means, materials and embodiments, perfusion shift measuring is not intended to be limited to the particulars disclosed; rather perfusion shift measuring extends to all functionally equivalent structures, methods, and uses such as are within the scope of the appended claims.

The illustrations of the embodiments described herein are intended to provide a general understanding of the structure of the various embodiments. The illustrations are not intended to serve as a complete description of all of the elements and features of the disclosure described herein. Many other embodiments may be apparent to those of skill in the art upon reviewing the disclosure. Other embodiments may be utilized and derived from the disclosure, such that structural and logical substitutions and changes may be made without departing from the scope of the disclosure. Additionally, the illustrations are merely representational and may not be drawn to scale. Certain proportions within the illustrations may be exaggerated, while other proportions may be minimized. Accordingly, the disclosure and the figures are to be regarded as illustrative rather than restrictive.

One or more embodiments of the disclosure may be referred to herein, individually and/or collectively, by the term "invention" merely for convenience and without intending to voluntarily limit the scope of this application to any particular invention or inventive concept. Moreover, although specific embodiments have been illustrated and described herein, it should be appreciated that any subsequent arrangement designed to achieve the same or similar purpose may be substituted for the specific embodiments shown. This disclosure is intended to cover any and all subsequent adaptations or variations of various embodiments. Combinations of the above embodiments, and other embodiments not specifically described herein, will be apparent to those of skill in the art upon reviewing the description.

The Abstract of the Disclosure is provided to comply with 37 C.F.R. §1.72(b) and is submitted with the understanding that it will not be used to interpret or limit the scope or meaning of the claims. In addition, in the foregoing Detailed Description, various features may be grouped together or described in a single embodiment for the purpose of streamlining the disclosure. This disclosure is not to be interpreted as reflecting an intention that the claimed embodiments require more features than are expressly recited in each claim. Rather, as the following claims reflect, inventive subject matter may be directed to less than all of the features of any of the disclosed embodiments. Thus, the following claims are incorporated into the Detailed Description, with each claim standing on its own as defining separately claimed subject matter.

The preceding description of the disclosed embodiments is provided to enable any person skilled in the art to practice the concepts described in the present disclosure. As such, the above disclosed subject matter is to be considered illustrative, and not restrictive, and the appended claims are intended to cover all such modifications, enhancements, and other embodiments which fall within the scope of the present disclosure. Thus, to the maximum extent allowed by law, the scope of the present disclosure is to be determined by the broadest permissible interpretation of the following claims and their equivalents and shall not be restricted or limited by the foregoing detailed description.

## Claims

1. A system for monitoring a patient, comprising:
a first sensor (110) adapted to be disposed at a central site, and adapted to provide a first signal for deriving perfusion index;
a second sensor (111) adapted to be disposed at a distal site, and adapted to provide a second signal for deriving perfusion index;
a processor (118);
a memory (116) that stores instructions, which when executed by the processor, cause the processor to:
correlate a first perfusion index based on the first signal from the first sensor and a second perfusion index based on the second signal from the second sensor;
determine a trend based analysis of perfusion comprising a perfusion shift based on the correlated first perfusion index and second perfusion index; and
determine the perfusion shift from a difference between a ratio of a current value of the first perfusion index to a current value of the second perfusion index, and a ratio of a past value of the first perfusion index to a past value of the second perfusion index.

2. The system of claim 1, wherein the trend based analysis further comprises a change in a cardiac output.

3. The system of claim 1 or 2, wherein the instructions further cause the processor (118) to:
determine the change in the cardiac output from a difference of a sum of a current value of the first perfusion index and a current value of the second perfusion index, and a sum of a past value of the first perfusion index and a past value of the second perfusion index.

4. The system of any of the foregoing claims, wherein the first perfusion indices are from the central site, and the second perfusion indices are from the distal site.

5. The system of any one of foregoing claims, wherein the trend based analysis comprises a plurality of changes in perfusion indices, or changes in cardiac output, or both, over a selected time period.

6. A patient monitor (112), comprising:
a display (120); and
the system of claims 1-5.

7. A tangible non-transitory computer readable storage medium that stores instructions, which, when executed by a processor (118), cause the processor to:
correlate a first perfusion index based on a first signal from a first sensor and a second perfusion index based on a second signal from a second sensor;
determine a trend based analysis of perfusion comprising a perfusion shift based on the correlated first perfusion index and second perfusion index; and
determine the perfusion shift from a difference between a ratio of a current value of the first perfusion index to a current value of the second perfusion index, and a ratio of a past value of the first perfusion index to a past value of the second perfusion index.

8. The tangible non-transitory computer readable storage medium of claim 7, wherein the instructions further cause the processor (118) to:
determine a change in a cardiac output from a difference of a sum of a current value of the first perfusion index and a current value of the second perfusion index, and a sum of a past value of the first perfusion index and a past value of the second perfusion index.

9. A computer-implemented method for monitoring a patient, the method comprising:
providing a first signal for deriving perfusion index from a first sensor (110) adapted to be disposed at a central site;
providing a second signal for deriving perfusion index from a second sensor (111) adapted to be disposed at a distal site;
correlating a first perfusion index based on the first signal from the first sensor and a second perfusion index based on the second signal from the second sensor;
determining a trend based analysis of perfusion comprising a perfusion shift based on the correlated first perfusion index and second perfusion index; and
determining the perfusion shift from a difference between a ratio of a current value of the first perfusion index to a current value of the second perfusion index, and a ratio of a past value of the first perfusion index to a past value of the second perfusion index.

10. The method of claim 9, wherein the trend based analysis comprises a cardiac output, and the method further comprises:
determining a change in the cardiac output from a difference of a sum of a current value of the first perfusion index and a current value of the second perfusion index, and a sum of a past value of the first perfusion index and a past value of the second perfusion index.

## Patentansprüche

1. System zum Überwachen eines Patienten, umfassend:
einen ersten Sensor (110), der dazu ausgelegt ist, an einer zentralen Stelle angeordnet zu werden, und dazu ausgelegt ist, ein erstes Signal zur Ableitung eines Perfusionsindex bereitzustellen;
einen zweiten Sensor (111), der dazu ausgelegt ist, an einer distalen Stelle angeordnet zu werden, und dazu ausgelegt ist, ein zweites Signal zur Ableitung eines Perfusionsindex bereitzustellen;
einen Prozessor (118);
einen Speicher (116), der Anweisungen speichert, die, wenn sie vom Prozessor ausgeführt werden, den Prozessor zu Folgendem veranlassen:
Korrelieren eines ersten Perfusionsindex basierend auf dem ersten Signal des ersten Sensors und eines zweiten Perfusionsindex basierend auf dem zweiten Signal des zweiten Sensors;
Bestimmen einer trendbasierten Analyse einer Perfusion, die eine Perfusionsverschiebung umfasst, basierend auf dem korrelierten ersten Perfusionsindex und zweiten Perfusionsindex; und
Bestimmen der Perfusionsverschiebung aus einer Differenz zwischen einem Verhältnis eines aktuellen Werts des ersten Perfusionsindex zu einem aktuellen Wert des zweiten Perfusionsindex und einem Verhältnis eines vergangenen Werts des ersten Perfusionsindex zu einem vergangenen Wert des zweiten Perfusionsindex.

2. System nach Anspruch 1, wobei die trendbasierte Analyse ferner eine Änderung eines Herzzeitvolumens umfasst.

3. System nach Anspruch 1 oder 2, wobei die Anweisungen den Prozessor (118) ferner zu Folgendem veranlassen:
Bestimmen der Änderung des Herzzeitvolumens aus einer Differenz einer Summe eines aktuellen Werts des ersten Perfusionsindex und einem aktuellen Wert des zweiten Perfusionsindex und einer Summe eines vergangenen Werts des ersten Perfusionsindex und einem vergangenen Wert des zweiten Perfusionsindex.

4. System nach einem der vorstehenden Ansprüche, wobei die ersten Perfusionsindizes von der zentralen Stelle stammen und die zweiten Perfusionsindizes von der distalen Stelle stammen.

5. System nach einem der vorstehenden Ansprüche, wobei die trendbasierte Analyse eine Vielzahl von Änderungen der Perfusionsindizes oder Änderungen des Herzzeitvolumens oder beides über einen ausgewählten Zeitraum umfasst.

6. Patientenmonitor (112), umfassend:
eine Anzeige (120); und
das System der Ansprüche 1-5.

7. Greifbares, nicht-transitorisches, computerlesbares Speichermedium, das Anweisungen speichert, die, wenn sie von einem Prozessor (118) ausgeführt werden, den Prozessor zu Folgendem veranlassen:
Korrelieren eines ersten Perfusionsindex basierend auf einem ersten Signal eines ersten Sensors und eines zweiten Perfusionsindex basierend auf einem zweiten Signal eines zweiten Sensors;
Bestimmen einer trendbasierten Analyse einer Perfusion, die eine Perfusionsverschiebung umfasst, basierend auf dem korrelierten ersten Perfusionsindex und zweiten Perfusionsindex; und
Bestimmen der Perfusionsverschiebung aus einer Differenz zwischen einem Verhältnis eines aktuellen Werts des ersten Perfusionsindex zu einem aktuellen Wert des zweiten Perfusionsindex und einem Verhältnis eines vergangenen Werts des ersten Perfusionsindex zu einem vergangenen Wert des zweiten Perfusionsindex.

8. Greifbares, nicht-transitorisches, computerlesbares Speichermedium nach Anspruch 7, wobei die Anweisungen den Prozessor (118) ferner zu Folgendem veranlassen:
Bestimmen einer Änderung eines Herzzeitvolumens aus einer Differenz einer Summe eines aktuellen Werts des ersten Perfusionsindex und einem aktuellen Wert des zweiten Perfusionsindex und einer Summe eines vergangenen Werts des ersten Perfusionsindex und einem vergangenen Wert des zweiten Perfusionsindex.

9. Computerimplementiertes Verfahren zum Überwachen eines Patienten, wobei das Verfahren Folgendes umfasst:
Bereitstellen eines ersten Signals zum Ableiten eines Perfusionsindex von einem ersten Sensor (110), der dazu ausgelegt ist, an einer zentralen Stelle angeordnet zu werden;
Bereitstellen eines zweiten Signals zum Ableiten eines Perfusionsindex von einem zweiten Sensor (111), der dazu ausgelegt ist, an einer distalen Stelle angeordnet zu werden;
Korrelieren eines ersten Perfusionsindex basierend auf dem ersten Signal des ersten Sensors und eines zweiten Perfusionsindex basierend auf dem zweiten Signal des zweiten Sensors;
Bestimmen einer trendbasierten Analyse einer Perfusion, die eine Perfusionsverschiebung umfasst, basierend auf dem korrelierten ersten Perfusionsindex und zweiten Perfusionsindex; und
Bestimmen der Perfusionsverschiebung aus einer Differenz zwischen einem Verhältnis eines aktuellen Werts des ersten Perfusionsindex zu einem aktuellen Wert des zweiten Perfusionsindex und einem Verhältnis eines vergangenen Werts des ersten Perfusionsindex zu einem vergangenen Wert des zweiten Perfusionsindex.

10. Verfahren nach Anspruch 9, wobei die trendbasierte Analyse ein Herzzeitvolumen umfasst, und das Verfahren ferner Folgendes umfasst:
Bestimmen einer Änderung des Herzzeitvolumens aus einer Differenz einer Summe eines aktuellen Werts des ersten Perfusionsindex und einem aktuellen Wert des zweiten Perfusionsindex und einer Summe eines vergangenen Werts des ersten Perfusionsindex und einem vergangenen Wert des zweiten Perfusionsindex.

## Revendications

1. Système de surveillance de patient, comprenant :
un premier capteur (110) adapté pour être disposé au niveau d'un site central et adapté pour fournir un premier signal permettant de dériver un indice de perfusion ;
un second capteur (111) adapté pour être disposé au niveau d'un site distal et adapté pour fournir un second signal pour dériver un indice de perfusion ;
un processeur (118),
une mémoire (116) qui stocke des instructions qui, lorsqu'elles sont exécutées par le processeur, amènent le processeur à :
corréler un premier indice de perfusion basé sur le premier signal du premier capteur et un second indice de perfusion basé sur le second signal du second capteur ;
déterminer une analyse de perfusion basée sur la tendance, comprenant un décalage de perfusion basé sur la corrélation entre le premier et le second indice de perfusion ; et
déterminer le décalage de perfusion à partir d'une différence entre un rapport entre une valeur actuelle du premier indice de perfusion et une valeur actuelle du second indice de perfusion, et un rapport entre une valeur passée du premier indice de perfusion et une valeur passée du second indice de perfusion.

2. Système selon la revendication 1, dans lequel l'analyse basée sur la tendance comprend en outre une modification du débit cardiaque.

3. Système selon la revendication 1 ou 2, dans lequel les instructions amènent en outre le processeur (118) à :
déterminer la modification du débit cardiaque à partir d'une différence entre une somme d'une valeur actuelle du premier indice de perfusion et une valeur actuelle du second indice de perfusion, et une somme d'une valeur passée du premier indice de perfusion et d'une valeur passée du second indice de perfusion.

4. Système selon l'une quelconque des revendications précédentes, dans lequel les premiers indices de perfusion proviennent du site central et les seconds indices de perfusion proviennent du site distal.

5. Système selon l'une quelconque des revendications précédentes, dans lequel l'analyse basée sur la tendance comprend une pluralité de modifications des indices de perfusion, ou une pluralité de modifications du débit cardiaque, ou les deux, sur une période de temps sélectionnée.

6. Moniteur de patient (112), comprenant :
un dispositif d'affichage (120) ; et
le système selon les revendications 1-5.

7. Support de stockage non transitoire tangible lisible par ordinateur stockant des instructions qui, lorsqu'elles sont exécutées par un processeur (118), amènent le processeur à :
corréler un premier indice de perfusion basé sur un premier signal provenant d'un premier capteur et un second indice de perfusion basé sur un second signal provenant d'un second capteur ;
déterminer une analyse de perfusion basée sur la tendance, comprenant un décalage de perfusion basé sur la corrélation entre le premier et le second indice de perfusion ; et
déterminer le décalage de perfusion à partir d'une différence entre un rapport entre une valeur actuelle du premier indice de perfusion et une valeur actuelle du second indice de perfusion, et un rapport entre une valeur passée du premier indice de perfusion et une valeur passée du second indice de perfusion.

8. Support de stockage non transitoire tangible lisible par ordinateur selon la revendication 7, dans lequel les instructions amènent le processeur (118) à :
déterminer une modification du débit cardiaque à partir d'une différence entre une somme d'une valeur actuelle du premier indice de perfusion et d'une valeur actuelle du second indice de perfusion, et une somme d'une valeur passée du premier indice de perfusion et d'une valeur passée du second indice de perfusion.

9. Procédé mis en œuvre par ordinateur pour surveiller un patient, le procédé comprenant :
la fourniture d'un premier signal pour dériver un indice de perfusion à partir d'un premier capteur (110) adapté pour être disposé au niveau d'un site central ;
la fourniture d'un second signal pour dériver un indice de perfusion à partir d'un second capteur (111) adapté pour être disposé au niveau d'un site distal ;
la corrélation d'un premier indice de perfusion basé sur le premier signal du premier capteur et d'un second indice de perfusion basé sur le second signal du second capteur ;
la détermination d'une analyse basée sur la tendance d'une perfusion comprenant un décalage de perfusion basé sur la corrélation entre le premier indice de perfusion et le second indice de perfusion ; et
la détermination du décalage de perfusion à partir d'une différence entre un rapport entre une valeur actuelle du premier indice de perfusion et une valeur actuelle du second indice de perfusion, et un rapport entre une valeur passée du premier indice de perfusion et une valeur passée du second indice de perfusion.

10. Procédé selon la revendication 9, dans lequel l'analyse basée sur la tendance comprend un débit cardiaque, et le procédé comprend en outre :
la détermination d'une modification du débit cardiaque à partir d'une différence entre une somme d'une valeur actuelle du premier indice de perfusion et une valeur actuelle du second indice de perfusion, et une somme d'une valeur passée du premier indice de perfusion et d'une valeur passée du second indice de perfusion.
